# EUROPEAN PATENT SPECIFICATION

(11) **EP 1 616 868 B1**
(45) Date of publication and mention of the grant of the patent: **12.09.2007**
(21) Application number: 04016493.1
(22) Date of filing: 13.07.2004
(51) Int. Cl.: C07D 305/14

(54) **Methods for obtaining paclitaxel from taxus plants**
Verfahren zur Gewinnung von Paclitaxel aus Taxuspflanzen
Méthode pour obtenir paclitaxes des plantes taxus

(43) Date of publication of application: 18.01.2006
(73) Proprietor: INDENA S.p.A., 20139 Milano (IT)
(72) Inventor: Gabetta, Bruno, 20139 Milano (IT); Zini, Gianfranco, 20139 Milano (IT)
(74) Representative: Minoja, Fabrizio

(56) References cited:
- EP-A- 0 553 780
- EP-B- 1 140 885
- WO-A-02/44162
- US-A- 5 744 333
- US-B1- 6 452 024

## Description

### FIELD OF THE INVENTION

This invention relates generally to methods for obtaining paclitaxel from plants containing paclitaxel. More particularly, the invention is directed to methods of extracting paclitaxel from plants of the *Taxaceae* family, which includes plants of the genus *Taxus,* such as *Taxus media,* involving a series of column chromatography steps followed by crystallization of paclitaxel.

### BACKGROUND OF THE INVENTION

Paclitaxel is an anticancer compound primarily derived from the bark of the Taxus brevifolia (Pacific yew) tree. In the 1960's, the National Cancer Institute began a study of plant extracts exhibiting anti-cancer or antineoplastic activities. The crude extract of bark from the Taxus brevifolia was found to inhibit a variety of tumors. In 1971, paclitaxel was isolated and described by M.C. Wani et al. (J. Am. Chem. Soc. 93, 2325, (1971)), who defined the structure of paclitaxel using chemical methods and X-ray crystallographic analysis. In 1979, Schiff and coworkers demonstrated paclitaxel's novel mechanism of action. This mechanism includes binding to microtubules and preventing their depolymerization under conditions where depolymerization would normally occur. Paclitaxel is currently used in the treatment of ovarian, breast and non-small cell lung cancers.

Although Paclitaxel is a natural product primarily extracted from the bark of the Pacific yew (*T*. *brevifolia*), it is also found in other members of the *Taxaceae* family including *T. canadensis* and *T. yunnanensis.* Paclitaxel is also present in the epigeal parts and roots of other yew species, including the European yew (*T. baccata*), whose needles contain paclitaxel and analogs, Asian yews (*T. wallichiana* and *T. chinensis*)*,* and yew trees cultivated for ornamental purposes. The following varieties of Taxus cultivars have also been found to contain paclitaxel: *T.x media "Henryi, " T.x media "Runyan," T. cuspidata, T.x media "Halloran, " T.x media "Hatfield," T.x media "Hicksii, " T.x media "Tauntonii, " T.x media "Dark Green Spreader, " T.x media "Wardii, " T.x media "Brownii, " T.x media "Densiformis, " T.x media "Nigra, " T.x cuspidata "Brevifolia,"* and *T. cuspidata "Spreader."*

All of these species contain paclitaxel in very limited amounts. For example, the bark of *T. brevifolia* and *T. yunnanensis* trees contains about 0.02% paclitaxel and the needles and roots of *T. media Hicksii* shrubs contain between about 0.005% to about 0.1% paclitaxel.

Therefore, it is of great interest to develop efficient methods of extracting high purity paclitaxel from plant material. The present invention provides methods for extracting high purity paclitaxel from plant material using chromatography followed by crystallization of the paclitaxel.

### SUMMARY OF THE INVENTION

Therefore, an object of the present invention is to provide methods for obtaining high purity paclitaxel from plants containing paclitaxel. These methods have the advantages of assuring the stability of paclitaxel, allowing the use of various Taxus cultivar or part of it, independent of the content of paclitaxel, and allowing the maximum recovery of the paclitaxel present in the biomass. The methods described in the present invention provide several advantages over previous methods. For example, it is known that paclitaxel can suffer degradation reactions such as epimerization in position 7, deacetylation in position 10, and detachment of the side chain in position 13. These degradation reactions, which affect both the quality and yield of the isolated paclitaxel, arise from heating, especially in media containing large amounts of alcoholic solvents. In the methods described in the present invention, alcohols are used in limited amounts and in diluted conditions and generally no heating is involved during the extraction process. Hence, the methods described herein enhance the stability of paclitaxel.

Furthermore, the methods described herein can use various types of plant parts as starting materials. More specifically, the starting material used in the present invention can be the leaves, stems, branches, bark, roots alone or mixtures thereof from a paclitaxel-containing plant. The starting material can be chosen independent of the ratio of the plant parts, the nature of the Taxus cultivar and the content of paclitaxel. The methods of the present invention provide the same process efficiency and assure a constant and high quality of paclitaxel. Also, the methods described herein allow for high yields of paclitaxel.

These methods comprise preparing a paclitaxel extract by extracting paclitaxel from a plant material. Paclitaxel is separated from the paclitaxel extract using column chromatography systems, wherein each column chromatography system comprises a stationary phase and an eluting solvent, and crystallizing the paclitaxel contained in at least one fraction obtained in the final chromatography step. In some embodiments, the paclitaxel is crystallized from all fractions obtained from the final chromatography step. The resulting paclitaxel crystals may be dried. As described above, several biomasses can be used as starting plant material. If Taxus media whole plants are used as biomass, the plant material is typically composed of about 40 to about 60 weight (w/w) percent aerial parts and about 60 to about 40 w/w percent roots. Additionally, the stationary phase and eluting solvent may differ between different systems.

In the methods of the present invention, paclitaxel is extracted from a plant material to form a paclitaxel extract. Paclitaxel may be extracted from the plant material using a solvent, such as a combination of water and an organic solvent. Column chromatography systems are used to separate the paclitaxel from the paclitaxel extract by obtaining at least one fraction containing paclitaxel after each chromatographic separation and, optionally, subjecting the at least one fraction to reduced pressure to remove the eluting solvent. When more than one fraction is collected following each separation, at least some of the fractions containing paclitaxel can be combined before removing the eluting solvent from this combination. Additionally, crystallizing may occur over a 24 hour period and it may occur at room temperature. The present invention further provides a method for extracting paclitaxel from *Taxus media* by extracting the plant material with an aqueous solution comprising about 40 to about 60 volume (v/v) percent acetone at room temperature.

In one embodiment, the methods of the present invention comprise preparing a paclitaxel extract by extracting paclitaxel from a plant material and then separating paclitaxel from the paclitaxel extract using a first column chromatography system comprising a first stationary phase and a first eluting solvent to obtain at least one fraction containing paclitaxel. Paclitaxel is then separated from the at least one fraction using a second column chromatography system comprising a second stationary phase and a second eluting solvent to obtain at least one fraction containing paclitaxel. Thereafter, paclitaxel is separated from the at least one fraction using a third column chromatography system comprising a third stationary phase and a third eluting solvent to obtain at least one fraction containing paclitaxel. Furthermore, paclitaxel is separated from the at least one fraction using a fourth column chromatography system comprising a fourth stationary phase and a fourth eluting solvent to remove at least cephalomannine and to obtain at least one fraction containing paclitaxel. The paclitaxel is then crystallized from the at least one fraction using a crystallizing solvent to obtain paclitaxel crystals.

In another embodiment, the methods of the present invention comprise preparing a paclitaxel extract by extracting paclitaxel from a plant material. Paclitaxel is then separated from the paclitaxel extract by using a first column chromatography system comprising a first stationary phase and a first eluting solvent to obtain at least one fraction containing paclitaxel and removing the first eluting solvent from the at least one fraction to form a first residue. Thereafter, paclitaxel is separated from the first residue by using a second column chromatography system comprising a second stationary phase and a second eluting solvent to obtain at least one fraction containing paclitaxel from the first residue, and then removing the second eluting solvent from the at least one fraction to form a second residue. Subsequently, paclitaxel is separated from the second residue by using a third column chromatography system comprising a third stationary phase and a third eluting solvent to obtain at least one fraction containing paclitaxel from the second residue, and then removing the third eluting solvent from the at least one fraction to form a third residue. Paclitaxel is then separated from the third residue by using a fourth column chromatography system comprising a fourth stationary phase and a fourth eluting solvent to obtain at least one fraction containing paclitaxel from the third residue, and then removing the fourth eluting solvent from the third residue to form a fourth residue. The paclitaxel is then crystallized from the fourth residue using a crystallizing solvent to obtain paclitaxel crystals.

In another embodiment, the methods of the present invention comprise preparing a plant material extract from the *Taxus media* by extracting the plant material with an aqueous solvent comprising about 40 to about 60 v/v percent acetone at room temperature and then preparing a paclitaxel extract by extracting paclitaxel from the plant material extract. Paclitaxel is then separated from the paclitaxel extract comprising using a first chromatography system comprising silica gel as a stationary phase and an eluting solvent comprising dichloromethane alone or a mixture of dichloromethane and methanol to obtain at least one fraction containing paclitaxel. The at least one fraction is then subjected to reduced pressure to remove the dichloromethane and/or methanol. Paclitaxel is then separated from the at least one fraction by using a second column chromatography system comprising neutral alumina as a stationary phase and as an eluting solvent comprising acetone to obtain at least one fraction containing paclitaxel, and then subjecting the at least one fraction to reduced pressure to remove the acetone. Thereafter, paclitaxel is separated from the at least one fraction by using a third column chromatography system comprising silica gel as a stationary phase and an eluting solvent comprising a mixture of n-hexane and acetone and to obtain at least one fraction containing paclitaxel, and then subjecting the at least one fraction to reduced pressure to remove the n-hexane and acetone. Moreover, paclitaxel is separated from the at least one fraction by using a fourth column chromatography system comprising silica gel as a stationary phase and an eluting solvent comprising t-butyl acetate to remove at least cephalomannine and to obtain at least one fraction containing paclitaxel, and subjecting the at least one fraction to reduced pressure to remove the t-butyl acetate. The at least one fraction is crystallized over a 24 hour period using a mixture of cyclohexane and acetone as the crystallizing solvent at room temperature to obtain paclitaxel crystals. Thereafter, the paclitaxel crystals are dried under vacuum for 48 hours.

In another embodiment, the methods of the present invention comprise preparing a plant material extract from the *Taxus media* by extracting the plant material with an aqueous solvent comprising about 40 to about 60 v/v percent acetone at room temperature and then preparing a paclitaxel extract by extracting paclitaxel from the plant material extract. Paclitaxel is then separated from the paclitaxel extract by using a first chromatography system comprising silica gel as a stationary phase and eluting solvent comprising dichloromethane and methanol to obtain at least one fraction containing paclitaxel and then subjecting the at least one fraction to reduced pressure to obtain a residue, and then dissolving the residue in acetone to obtain an acetone-residue composition. Subsequently, paclitaxel is separated from the acetone-residue composition by using a second column chromatography system comprising neutral alumina as a stationary phase and an eluting solvent comprising acetone to obtain at least one fraction containing paclitaxel. The at least one fraction is subjected to reduced pressure to remove the acetone. Paclitaxel is then separated from the at least one fraction by using a third column chromatography system comprising silica gel as a stationary phase and an eluting solvent comprising n-hexane and acetone to obtain at least one fraction containing paclitaxel, and subjecting the at least one fraction to reduced pressure to remove the n-hexane and acetone. Paclitaxel is separated from the at least one fraction by using a fourth column chromatography system comprising silica gel as a stationary phase and an eluting solvent comprising t-butyl acetate to remove at least cephalomannine and to obtain at least one fraction containing paclitaxel, and subjecting the at least one fraction to reduced pressure to remove the t-butyl acetate. The at least one fraction is crystallized over a 24 hour period using a mixture of cyclohexane and acetone as the crystallizing solvent at room temperature to obtain paclitaxel crystals. The paclitaxel crystals are dried under vacuum for 48 hours.

In one embodiment, the methods of the present invention comprise preparing a plant material extract from the *Taxus media* by extracting the plant material with an aqueous solution comprising about 40 to about 60 v/v percent acetone at room temperature and preparing a paclitaxel extract by extracting paclitaxel from the plant material extract. Paclitaxel is separated from the paclitaxel extract by using a first chromatography system comprising neutral alumina as a stationary phase and an eluting solvent comprising dichloromethane and methanol to obtain at least one fraction containing paclitaxel and subjecting the at least one fraction to reduced pressure to remove the dichloromethane and methanol. The paclitaxel is separated from the at least one fraction by using a second column chromatography system comprising silica gel as a stationary phase and an eluting solvent comprising dichloromethane and methanol to obtain at least one fraction containing paclitaxel, and subjecting the at least one fraction to reduced pressure to obtain a residue, and dissolving the residue in acetone to obtain an acetone-residue composition. Thereafter, paclitaxel is separated from the acetone-residue composition by using a third column chromatography system comprising silica gel as a stationary phase and an eluting solvent comprising n-hexane and acetone to obtain at least one fraction containing paclitaxel, and subjecting the at least one fraction to reduced pressure to remove the mixture of n-hexane and acetone. Subsequently, paclitaxel is separated from the at least one fraction by using a fourth column chromatography system comprising silica gel as a stationary phase and an eluting solvent comprising a t-butyl acetate to remove at least cephalomannine and to obtain at least one fraction containing paclitaxel, and subjecting the at least one fraction to reduced pressure to remove the t-butyl acetate. Thereafter, the at least one fraction is crystallized over a 24 hour period using a mixture of cyclohexane and acetone as the crystallizing solvent at room temperature to obtain paclitaxel crystals. The paclitaxel crystals are dried under vacuum for 48 hours.

### BRIEF DESCRIPTION OF THE DRAWING

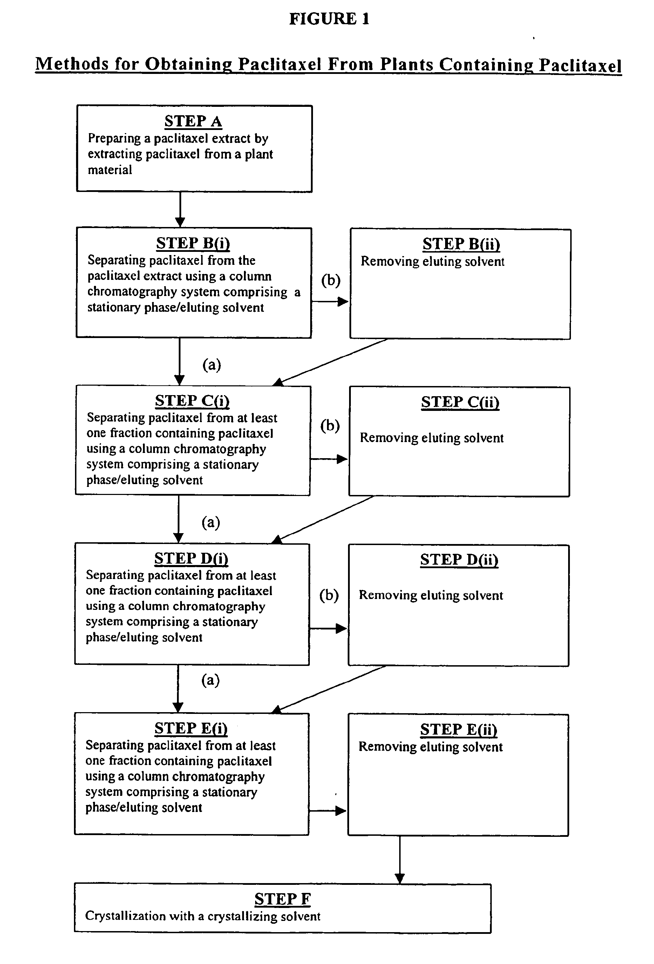

Figure 1 is a flow diagram outlining an embodiment of the methods of the present invention.

### DETAILED DESCRIPTION OF THE INVENTION

The methods of the present invention include a method for obtaining paclitaxel from plants containing paclitaxel. Figure 1 shows a flow diagram outlining steps of an embodiment of the methods of the present invention. **Step A** comprises preparing a paclitaxel extract by extracting paclitaxel from a plant material containing paclitaxel. The paclitaxel is then separated from the other constituents in the paclitaxel extract in **Steps B - E** using column chromatography separation systems. Each step marked with **(i)** involves separating paclitaxel from the paclitaxel extract or chromatographic fraction using a column chromatography system comprising a stationary phase and an eluting solvent to obtain at least one fraction containing paclitaxel. Each step marked with **(ii)** comprises removing the eluting solvent from the at least one fraction obtained in the previous chromatography step. In a preferred embodiment, the at least one fraction is subjected to reduced pressure to remove the eluting solvent. Labels **(a)** and **(b)** in Figure 1 designate alternative separation routes. For instance, if route **(a)** is followed for each separation step, the eluting solvent is not removed from the at least one fraction of paclitaxel. In contrast, if route **(b)** is used for each separation step, the eluting solvent is removed from the at least one fraction of paclitaxel. In certain embodiments, route **(a)** can be followed for certain of the separation steps and route **(b)** can be followed for other separation steps. For example in **Step B,** route **(b)** can be taken so that the eluting solvent is removed from the fraction obtained in **Step B** whereas in **Step C,** route **(a)** is taken so that the fraction is directly subjected to separation in **Step D,** without removal of the eluting solvent of **Step C. Step F** involves crystallizing the at least one fraction obtained from the final chromatography step. In a specific embodiment, the at least one fraction obtained from **Step E(ii)** is crystallized in **Step F.** Although only four chromatography steps are shown in Figure 1, there can be additional chromatography steps before crystallization. The crystallization step can be followed by a drying step.

### The Plant Material

The starting material for use in Step A of the present invention is plant material containing paclitaxel. Suitable plant material can be obtained from the plant parts of the *Taxaceae* family, *i.e. Taxus* plant. Preferably, the plant material is prepared from *Taxus media.* Suitable varieties of *Taxus media* include *T.x media "Hill, " T.x media "Henryi, " T.x media "Runyan," T. cuspidata, T.x media "Halloran," T.x media "Hatfield," T.x media "Hicksii, " T.x media "Tauntonii," T.x media "Dark Green Spreader, " T.x media "Wardii, " T.x media "Brownii, " T.x media "Densiformis, " T.x media "Nigra," T.x cuspidata "Brevifolia," and T.x cuspidata "Spreader."* Further, the plant material can be prepared from various parts of *a Taxus* plant, such as fresh or dry roots, leaves, branches, seeds, bark, stems or mixtures thereof. Preferably, the plant material is prepared from about 40 to about 60 w/w percent aerial parts and about 60 to about 40 w/w percent roots. The plant material is obtained from a paclitaxel-containing plant part by, for example, extracting, crushing or cutting. In a specific embodiment, the plant material to be extracted is ground using a blade milling line, equipped, for instance, with a 10 mm diameter net. In one embodiment, the plant material is extracted using at least one extraction solvent. Suitable solvents include aqueous solvents, such as, aqueous organic solvents, e.g. acetone in water. Preferably, a mixture of about 40 to about 60 volume percent acetone and about 60 to about 40 volume percent water is used. Aqueous acetone can prevent degradation of the paclitaxel. Preferably, extraction takes place at room temperature. Extraction at room temperature can prevent degradation of paclitaxel.

### Extraction of Paclitaxel and Cogeners

Prior to the first chromatography step, e.g. **Step B,** an extract of paclitaxel is prepared such as in **Step A.** In one embodiment, the paclitaxel extract is prepared by extracting the paclitaxel from the plant material with a solvent. In a specific embodiment, the paclitaxel extract is prepared by treating an aqueous acetone extract of the plant material. Suitable extraction solvents include without limitation aliphatic esters, such as ethyl acetate, or chlorinated solvents, such as chloroform or dichloromethane. A preferred extraction solvent is dichloromethane. In a specific embodiment, before the paclitaxel is extracted with one of the above solvents, the aqueous acetone extract is concentrated under vacuum to eliminate the acetone. The concentrate can then be diluted with a solvent. This dilution solvent can include methyl alcohol or other hydrosoluble alcoholic solvents such as ethyl or propyl alcohol. Paclitaxel is then extracted from the diluted concentrate using an extraction solvent such as dichloromethane and the other extraction solvents discussed above. A paclitaxel extract is formed.

In one embodiment, the paclitaxel extract is concentrated, under vacuum, in order to eliminate the solvents. The concentrated paclitaxel extract contains paclitaxel and its cogeners, chlorophylls, fats, lignans, flavonoids, phenols and various polar impurities. Cogeners include, for example, cephalomannine, N-debenzoyl-N-hexanoyl-paclitaxel, N-debenzoyl-N-hexanoyl-N-methyl-paclitaxel, N-debenzoyl-N-phenylacetyl-paclitaxel, N-debenzoyl-N-cinnamoyl-paclitaxel, and 2-debenzoyl-2-tigloyl-paclitaxel. Each of these may be accompanied by the respective 7-epi, 10-deacetyl and 7-0-xylosyl derivative. The corresponding latter derivatives of paclitaxel are also present. These non-paclitaxel constituents or impurities can be removed using chromatography techniques as discussed below.

### Purification of Paclitaxel by Chromatography

Paclitaxel is separated from the paclitaxel extract by the application of at least 4 column chromatography steps designated in Figure 1 as **Steps B - E. Steps B(i) - E(i)** each include the use of a chromatography system comprising a stationary phase and an eluting solvent. Each system can be the same apparatus with a different stationary phase and eluting solvent. Suitable stationary phases include without limitation neutral alumina and silica-gel. Possible eluting solvents include without limitation acetone, dichloromethane, methanol, n-hexane, t-butyl acetate, chloroform, and ethyl acetate.

Each chromatography step includes using a column chromatography system comprising a stationary phase and an eluting solvent to obtain at least one fraction containing paclitaxel. At least one fraction may include a combination of multiple fractions obtained from a chromatography step. For instance, more than one fraction can be obtained from a chromatography step. These fractions are then combined and the next chromatography step is performed on the combined fractions.

In one embodiment, the eluting solvent is removed from at least one fraction. At least one fraction may be subjected to reduced pressure in **Steps B(ii) - E(ii)** to remove the eluting solvent. Removing the solvent can include just a portion of the solvent, not necessarily all the solvent in the at least one fraction.

In one embodiment, the first chromatography step, **Step B(i),** generally separates fats, chlorophylls, low-molecular weight phenolics and very polar impurities from the paclitaxel. The second chromatography step, **Step C(i),** generally separates additional impurities, in particular flavonoids and lignans from the paclitaxel. The third chromatography step, **Step D(i),** generally separates other taxanes from paclitaxel. Such taxanes include the above-mentioned taxane derivatives, in particular, cephalomannine and minor amounts of the other taxanes, such as those discussed above. The fourth chromatography step, **Step E(i),** separates the cogener cephalomannine from the paclitaxel. Suitable stationary phases for removing cephalomannine include silica gel. Examples of useful eluting solvents include t-butyl acetate, i-butyl acetate, n-butyl formate, i-butyl formate, t-butyl formate, and s-butyl formate. *See* U.S. Patent No. 6,333,419, the contents of which are herein incorporated by reference in their entirety.

In one embodiment, the first column chromatography system comprises a stationary phase comprising silica gel and an eluting solvent comprising methylene chloride followed by a mixture of methylene chloride-methanol in 99:1 or 98:2 v/v ratio. In another embodiment, the second column chromatography system comprises a stationary phase comprising neutral alumina and an eluting solvent comprising acetone. In yet another embodiment, the third column chromatography system comprises a stationary phase comprising silica gel and an eluting solvent comprising n-hexane and acetone in a ratio of 4:1 v/v. In another embodiment, the fourth column chromatography system comprises a stationary phase comprising silica gel and an eluting solvent comprising t-butyl acetate.

Optionally, it is also possible to eliminate the impurities in a different order. For example, the removal of phenolics over alumina can be performed before the elimination of chlorophylls and fats using silica gel as the stationary phase.

### Crystallization of Paclitaxel

Following the final chromatography step, the at least one fraction containing paclitaxel that is obtained from that step is crystallized in **Step F** using a crystallizing solvent to obtain paclitaxel crystals. Suitable crystallizing solvents include cyclohexane, acetone, n-hexane, i-hexane, n-heptane, t-butyl acetate and mixtures thereof. Preferably, a mixture of cyclohexane and acetone in a ratio of 1:1 is used. In one embodiment, the crystallization occurs at room temperature. Generally, the crystallization occurs over a 24 hour period. However, crystallization can also occur over other time periods. Following crystallization, the paclitaxel crystals are then dried under vacuum at a temperature, such as between about 50°C and about 60°C. Preferably, the crystals are dried for about 48 hours.

### EXAMPLE 1

One ton of dried and ground plant material obtained from the *T. media Hicksii* composed of about 500 kg roots and about 500 kg leaves and twigs was extracted at room temperature with 15.000 L of 50% aqueous acetone. The extract was concentrated under vacuum to about 300 L. Then, 150 L of methanol was added and five extractions with 200 L of dichloromethane were carried out. The pooled organic layers were concentrated under vacuum until a soft residue was obtained, which was redissolved in dichloromethane and column chromatographed over 180 kg of silica gel. About 2.700 L of dichloromethane were eluted through the column and discarded.

Paclitaxel was then eluted with a mixture of dichloromethane-methanol 99:1 (v/v). The solution was then evaporated under vacuum in order to obtain a soft residue. The residue was dissolved in 8 L of acetone to obtain an acetone-residue which was passed through a column containing 30 kg of neutral alumina, eluting with the same solvent. Fractions containing paclitaxel were pooled and concentrated under vacuum to 8 L.

The acetone solution was then charged on a column containing 180 kg of silica gel packed with the mixture of n-hexane-acetone 4:1. Elution with this eluent provided paclitaxel free of most of the other taxanes, except cephalomannine. Fractions containing paclitaxel were pooled and concentrated under vacuum to 5 L.

The solution was then chromatographed over 180 kg of silica gel packed with t-butyl acetate, eluting with this solvent. Fractions containing paclitaxel were pooled and evaporated under vacuum until a soft residue was obtained. Further elution of the columns with t-butyl acetate removed at least cephalomannine. The soft residue (about 300 g) containing paclitaxel was dissolved in 2 L of acetone, diluted with 1.2 L of cyclohexane and left to crystallize. After filtration and drying at 50°C for 48 hours, 255 g of paclitaxel having a purity greater than 99% were obtained.

### EXAMPLE 2

One hundred kilograms of dried and ground plant material from Taxus media dark green spreader whole plant composed of about 40 kg roots and about 60 kg leaves and twigs were extracted at room temperature with 1,500 L of 50% aqueous acetone. The extract was concentrated under vacuum to 30 L. Then, 15 L of methanol was added and five extractions with 20 L of dichloromethane were carried out. The dichloromethane extracts were pooled and concentrated under vacuum to 15 L.

Methanol (150 ml) was added and the solution was passed through a column containing 13 kg of neutral alumina eluting with dichloromethane-methanol 99:1 v/v. Fractions containing paclitaxel were collected and concentrated under vacuum to 5 L.

The concentrated solution was charged on a column containing 9 kg of silica gel and eluted with dichloromethane-methanol 99:1 v/v. After the elution of polar impurities, 220 L of fractions containing paclitaxel were collected, pooled and evaporated under vacuum in order to obtain a soft residue (190 g).

The residue was dissolved in 400 ml of acetone to form an acetone-residue which was column chromatographed over 9 kg of silica gel eluting with n-hexane-acetone 4:1. Fractions containing paclitaxel were collected and purified again by column chromatography over silica gel eluting with t-butyl acetate, as described in Example 2. After crystallization from the mixture with cyclohexane-acetone 1:1 and drying at 55°C, 18.5 g of paclitaxel having a purity greater than 99% were obtained.

## Claims

1. A method for obtaining paclitaxel from plants containing paclitaxel comprising:
(a) preparing a paclitaxel extract by extracting paclitaxel from a plant material;
(b) separating paclitaxel from the paclitaxel extract using a first column chromatography system comprising a first stationary phase and a first eluting solvent to obtain at least one fraction containing paclitaxel;
(c) separating paclitaxel from the at least one fraction obtained in step (b) using a second column chromatography system comprising a second stationary phase and a second eluting solvent to obtain at least one fraction containing paclitaxel;
(d) separating paclitaxel from the at least one fraction obtained in step (c) using a third column chromatography system comprising a third stationary phase and a third eluting solvent to obtain at least one fraction containing paclitaxel;
(e) separating paclitaxel from the at least one fraction obtained in step (d) using a fourth column chromatography system comprising a fourth stationary phase and a fourth eluting solvent to remove at least cephalomannine and to obtain at least one fraction containing paclitaxel; and
(f) crystallizing the paclitaxel contained in the at least one fraction obtained in step (e) using a crystallizing solvent to obtain paclitaxel crystals.

2. The method of claim 1, further comprising drying the paclitaxel crystals obtained in step (f).

3. The method of claim 1, wherein the plant material is obtained from plant parts.

4. The method of claim 3, wherein the plant material extract is prepared by extracting the plant material comprising 40 to 60 w/w percent aerial parts and 60 to 40 w/w percent roots.

5. The method of claim 1, wherein the plant material comprises plant material obtained from *Taxus media, T.x media "Henryi," T.x media "Runyan," T. cuspidata, T.x media "Halloran, " T.x media "Hatfield, " T.x media "Hicksii, " T.x media "Tauntonii, " T.x media "Dark Green Spreader, " T.x media "Wardii, " T.x media "Brownii,* " *T.x media "Densiformis, " T.x media "Nigra," T.x cuspidata "Brevifolia, " or T. cuspidata "Spreader ".*

6. The method of claim 1, wherein the second stationary phase comprises neutral alumina.

7. The method of claim 1, wherein the first, third, or fourth stationary phase comprises silica gel.

8. The method of claim 1, wherein the first, second or third eluting solvent comprises acetone, dichloromethane, methanol, n-hexane, t-butyl acetate, chloroform or ethyl acetate.

9. The method of claim 1, wherein the first eluting solvent comprises dichloromethane and methanol.

10. The method of claim 1, wherein the second eluting solvent comprises acetone.

11. The method of claim 1, wherein the third eluting solvent comprises n-hexane and acetone.

12. The method of claim 1, wherein the fourth eluting solvent comprises t-butyl acetate, i-butyl acetate, n-butyl formate, i-butyl formate, t-butyl formate, or s-butyl formate.

13. The method of claim 1, wherein the crystallizing solvent comprises cyclohexane and acetone.

14. A method for obtaining paclitaxel from plants containing paclitaxel comprising:
(a) preparing a paclitaxel extract by extracting paclitaxel from a plant material;
(b) separating paclitaxel from the paclitaxel extract comprising:
(i) using a first column chromatography system comprising a first stationary phase and a first eluting solvent to obtain at least one fraction containing paclitaxel and
(ii) removing the first eluting solvent from the at least one fraction obtained in step (b)(i) to form a first residue;
(c) separating paclitaxel from the first residue obtained in step (b)(ii) comprising:
(i) using a second column chromatography system comprising a second stationary phase and a second eluting solvent to obtain at least one fraction containing paclitaxel from the first residue, and
(ii) removing the second eluting solvent from the at least one fraction obtained in step (c)(i) to form a second residue;
(d) separating paclitaxel from the second residue obtained in step (c)(ii) comprising:
(i) using a third column chromatography system comprising a third stationary phase and a third eluting solvent to obtain at least one fraction containing paclitaxel from the second residue, and
(ii) removing the third eluting solvent from the at least one fraction obtained in step (d)(i) to form a third residue;
(e) separating paclitaxel from the third residue obtained in step (d)(ii) comprising:
(i) using a fourth column chromatography system comprising a fourth stationary phase and a fourth eluting solvent to obtain at least one fraction containing paclitaxel from the third residue, and
(ii) removing the fourth eluting solvent from the third residue obtained in step (e)(i) to form a fourth residue; and
(f) crystallizing paclitaxel from the fourth residue obtained in step (e)(ii) using a crystallizing solvent to obtain paclitaxel crystals.

15. The method of claim 14 wherein in step (e) at least cephalomannine is removed from the third residue.

16. The method of claim 14, wherein the first eluting solvent is removed by subjecting the at least one fraction obtained in step (b)(i) to reduced pressure.

17. The method of claim 14, wherein the second eluting solvent is removed by subjecting the at least one fraction obtained in step (c)(i) to reduced pressure.

18. The method of claim 14, wherein the third eluting solvent is removed by subjecting the at least one fraction obtained in step (d)(i) to reduced pressure.

19. The method of claim 14, wherein the fourth eluting solvent is removed by subjecting the at least one fraction obtained in step (e)(i) to reduced pressure.

20. The method of claim 14, further comprising drying the paclitaxel crystals obtained in step (f).

21. The method of claim 20, wherein the paclitaxel crystals are dried under vacuum.

22. The method of claim 14, wherein the paclitaxel is extracted from the plant material using a first aqueous solvent.

23. The method of claim 22, wherein the first aqueous solvent comprises water and acetone.

24. The method of claim 14, wherein step (a) occurs at room temperature.

25. The method of claim 14, wherein more than one fraction is obtained in step (b)(i) and at least some of the fractions are combined before removing the first eluting solvent in step (b)(ii).

26. The method of claim 14, wherein more than one fraction is obtained in step (c)(i) and at least some of the fractions are combined before removing the second eluting solvent in step (c)(ii).

27. The method of claim 14, wherein more than one fraction is obtained in step (d)(i) and at least some of the fractions are combined before removing the third eluting solvent in step (d)(ii).

28. The method of claim 14, wherein more than one fraction is obtained in step (e)(i) and at least some of the fractions are combined before removing the fourth eluting solvent in step (e)(ii).

29. The method of claim 14, wherein the crystallizing of step (f) occurs over a 24 hour period.

30. The method of claim 14, wherein the crystallizing of step (f) occurs at room temperature.

31. The method of claim 14, wherein the plant material is obtained from plant parts.

32. The method of claim 31, wherein the plant material extract is prepared by extracting the plant material from plant parts comprising about 40 to about 60 w/w percent aerial parts and about 60 to about 40 w/w percent roots.

33. The method of claim 14, wherein the plant material comprises plant material obtained from *Taxus media, T.x media "Henryi, " T.x media "Runyan," T. cuspidata, T.x media "Halloran, " T.x media "Hatfield," T.x media "Hicksii, " T.x media "Tauntonii, " T.x media "Dark Green Spreader, " T.x media "Wardii, " T.x media "Brownii, " T.x media "Densiformis, " T.x media "Nigra, " T.x cuspidata "Brevifolia, " or T. cuspidata "Spreader ".*

34. The method of claim 14, wherein the second stationary phase comprises neutral alumina.

35. The method of claim 14, wherein the first, third, or fourth stationary phase comprises silica gel.

36. The method of claim 14, wherein the first, second or third eluting solvent comprises acetone, dichloromethane, methanol, n-hexane, t-butyl acetate, chloroform, or ethyl acetate.

37. The method of claim 14, wherein the first eluting solvent comprise dichloromethane and methanol.

38. The method of claim 14, wherein the second eluting solvent comprise acetone.

39. The method of claim 14, wherein the third eluting solvent comprise n-hexane, and acetone.

40. The method of claim 14, wherein the fourth eluting solvent comprises t-butyl acetate, i-butyl acetate, n-butyl formate, i-butyl formate, t-butyl formate, or s-butyl formate.

41. The method of claim 14, wherein the crystallizing solvent comprises cyclohexane and acetone.

42. A method for obtaining paclitaxel from *Taxus media* comprising:
(a) preparing a plant material extract from the *Taxus media* by extracting the plant material with an aqueous solvent comprising 40 to 60 v/v percent acetone at room temperature;
(b) preparing a paclitaxel extract by extracting paclitaxel from the plant material extract obtained in (a);
(c) separating paclitaxel from the paclitaxel extract comprising:
(i) using a first chromatography system comprising silica gel as a stationary phase and an eluting solvent comprising dichloromethane alone or a mixture of dichloromethane and methanol to obtain at least one fraction containing paclitaxel and
(ii) subjecting the at least one fraction obtained in step (c)(i) to reduced pressure to remove the dichloromethane and/or methanol;
(d) separating paclitaxel from the at least one fraction obtained in step (c)(ii) comprising:
(i) using a second column chromatography system comprising neutral alumina as a stationary phase and as an eluting solvent comprising acetone to obtain at least one fraction containing paclitaxel, and
(ii) subjecting the at least one fraction obtained in step (d)(i) to reduced pressure to remove the acetone;
(e) separating paclitaxel from the at least one fraction obtained in step (d)(ii) comprising:
(i) using a third column chromatography system comprising silica gel as a stationary phase and an eluting solvent comprising a mixture of n-hexane and acetone and to obtain at least one fraction containing paclitaxel, and
(ii) subjecting the at least one fraction obtained in step (e)(i) to reduced pressure to remove the n-hexane and acetone;
(f) separating paclitaxel from the at least one fraction obtained in step (e)(ii) comprising:
(i) using a fourth column chromatography system comprising silica gel as a stationary phase and an eluting solvent comprising t-butyl acetate to remove at least cephalomannine and to obtain at least one fraction containing paclitaxel, and
(ii) subjecting the at least one fraction obtained in step (f)(i) to reduced pressure to remove the t-butyl acetate;
(g) crystallizing the at least one fraction obtained in step (f)(ii) over a 24 hour period using a mixture of cyclohexane and acetone as the crystallizing solvent at room temperature to obtain paclitaxel crystals; and
(h) drying the paclitaxel crystals obtained in (g) under vacuum for 48 hours.

43. The method of claim 42, wherein the preparation of the paclitaxel extract in step (b) further comprises:
(a) concentrating the plant material extract under reduced pressure to remove the acetone to form a concentrate;
(b) diluting the concentrate with methyl alcohol;
(c) extracting the concentrate with dichloromethane to form a dichloromethane extract; and
(d) concentrating the dichloromethane extract under reduced pressure to form the paclitaxel extract.

44. A method for obtaining paclitaxel from *Taxus media* comprising:
(a) preparing a plant material extract from the *Taxus media* by extracting the plant material with an aqueous solvent comprising about 40 to about 60 v/v percent acetone at room temperature;
(b) preparing a paclitaxel extract by extracting paclitaxel from the plant material extract obtained in (a);
(c) separating paclitaxel from the paclitaxel extract comprising:
(i) using a first chromatography system comprising silica gel as a stationary phase and eluting solvent comprising dichloromethane and methanol to obtain at least one fraction containing paclitaxel,
(ii) subjecting the at least one fraction obtained in step (c)(i) to reduced pressure to obtain a residue, and
(iii) dissolving the residue obtained in step (c)(ii) in acetone to obtain an acetone-residue composition;
(d) separating paclitaxel from the acetone-residue composition obtained in step (c)(iii) comprising:
(i) using a second column chromatography system comprising neutral alumina as a stationary phase and an eluting solvent comprising acetone to obtain at least one fraction containing paclitaxel, and
(ii) subjecting the at least one fraction obtained in step (d)(i) to reduced pressure to remove the acetone;
(e) separating paclitaxel from the at least one fraction obtained in step (d)(ii) comprising:
(i) using a third column chromatography system comprising silica gel as a stationary phase and an eluting solvent comprising n-hexane and acetone to obtain at least one fraction containing paclitaxel, and
(ii) subjecting the at least one fraction obtained in step (e)(i) to reduced pressure to remove the n-hexane and acetone;
(f) separating paclitaxel from the at least one fraction obtained in step (e)(ii) comprising:
(i) using a fourth column chromatography system comprising silica gel as a stationary phase and an eluting solvent comprising t-butyl acetate to remove at least cephalomannine and to obtain at least one fraction containing paclitaxel, and
(ii) subjecting the at least one fraction obtained in step (f)(i) to reduced pressure to remove the t-butyl acetate;
(g) crystallizing the at least one fraction obtained in step (f)(ii) over a 24 hour period using a mixture of cyclohexane and acetone as the crystallizing solvent at room temperature to obtain paclitaxel crystals; and
(h) drying the paclitaxel crystals obtained in (g) under vacuum for 48 hours.

45. The method of claim 44 wherein the *Taxus media* comprises *T. media Hicksii.*

46. A method for obtaining paclitaxel from *Taxus media* comprising:
(a) preparing a plant material extract from the *Taxus media* by extracting the plant material with an aqueous solution comprising about 40 to about 60 v/v percent acetone at room temperature;
(b) preparing a paclitaxel extract by extracting paclitaxel from the plant material extract obtained in (a);
(c) separating paclitaxel from the paclitaxel extract comprising:
(i) using a first chromatography system comprising neutral alumina as a stationary phase and an eluting solvent comprising dichloromethane and methanol to obtain at least one fraction containing paclitaxel and
(ii) subjecting the at least one fraction obtained in step (c)(i) to reduced pressure to remove the dichloromethane and methanol;
(d) separating paclitaxel from the at least one fraction obtained in step (c)(ii) comprising:
(i) using a second column chromatography system comprising silica gel as a stationary phase and an eluting solvent comprising dichloromethane and methanol to obtain at least one fraction containing paclitaxel,
(ii) subjecting the at least one fraction obtained in step (d)(i) to reduced pressure to obtain a residue, and
(iii) dissolving the residue obtained in step (d)(ii) in acetone to obtain an acetone-residue composition;
(e) separating paclitaxel from the acetone-residue composition obtained in step (d)(iii) comprising:
(i) using a third column chromatography system comprising silica gel as a stationary phase and an eluting solvent comprising n-hexane and acetone to obtain at least one fraction containing paclitaxel, and
(ii) subjecting the at least one fraction obtained in step (e)(i) to reduced pressure to remove the mixture of n-hexane and acetone;
(f) separating paclitaxel from the at least one fraction obtained in step (e)(ii) comprising:
(i) using a fourth column chromatography system comprising silica gel as a stationary phase and an eluting solvent comprising a t-butyl acetate to remove at least cephalomannine and to obtain at least one fraction containing paclitaxel, and
(ii) subjecting the at least one fraction obtained in step (f)(i) to reduced pressure to remove the t-butyl acetate;
(g) crystallizing the at least one fraction obtained in step (f)(ii) over a 24 hour period using a mixture of cyclohexane and acetone as the crystallizing solvent at room temperature to obtain paclitaxel crystals; and
(h) drying the paclitaxel crystals obtained in (g) under vacuum for 48 hours.

47. The method of claim 46, wherein the *Taxus media* comprises *T. media "dark green spreader* ".

## Patentansprüche

1. Verfahren zum Gewinnen von Paclitaxel aus Paclitaxel enthaltenden Pflanzen, umfassend:
(a) Herstellen eines Paclitaxelextrakts durch Extrahieren von Paclitaxel aus einem Pflanzenmaterial;
(b) Abtrennen von Paclitaxel aus dem Paclitaxelextrakt unter Verwendung eines ersten Säulenchromatographiesystems, umfassend eine erste stationäre Phase und ein erstes Elutionslösungsmittel, um mindestens eine Paclitaxel enthaltende Fraktion zu erhalten;
(c) Abtrennen von Paclitaxel aus der mindestens einen in Schritt (b) erhaltenen Fraktion unter Verwendung eines zweiten Säulenchromatographiesystems, umfassend eine zweite stationäre Phase und ein zweites Elutionslösungsmittel, um mindestens eine Paclitaxel enthaltende Fraktion zu erhalten;
(d) Abtrennen von Paclitaxel aus der mindestens einen in Schritt (c) erhaltenen Fraktion unter Verwendung eines dritten Säulenchromatographiesystems, umfassend eine dritte stationäre Phase und ein drittes Elutionslösungsmittel, um mindestens eine Paclitaxel enthaltende Fraktion zu erhalten;
(e) Abtrennen von Paclitaxel aus der mindestens einen in Schritt (d) erhaltenen Fraktion unter Verwendung eines vierten Säulenchromatographiesystems, umfassend eine vierte stationäre Phase und ein viertes Elutionslösungsmittel, um mindestens Cephalomannin zu entfernen und um mindestens eine Paclitaxel enthaltende Fraktion zu erhalten; und
(f) Kristallisieren des in der mindestens einen in Schritt (e) erhaltenen Fraktion enthaltenen Paclitaxels unter Verwendung eines Kristallisationslösungsmittels, um Paclitaxelkristalle zu erhalten.

2. Verfahren nach Anspruch 1, weiterhin umfassend Trocknen der in Schritt (f) erhaltenen Paclitaxelkristalle.

3. Verfahren nach Anspruch 1, wobei das Pflanzenmaterial aus Pflanzenteilen erhalten wird.

4. Verfahren nach Anspruch 3, wobei der Pflanzenmaterialextrakt durch Extrahieren des Pflanzenmaterials, umfassend 40 bis 60 Gewicht / Gewicht Prozent oberirdische Teile und 60 bis 40 Gewicht / Gewicht Prozent Wurzeln, hergestellt wird.

5. Verfahren nach Anspruch 1, wobei das Pflanzenmaterial Pflanzenmaterial umfasst, das aus *Taxus media, T.x media "Henryi", T. x media "Runyan", T. cuspidata, T. x media "Halloran", T. x media "Hatfield", T. x media "Hicksii", T. x media "Tauntonii ", T. x media "Dark Green Spreader", T. x media "Wardii", T.x media "Brownii", T.x media "Densiformis", T.x media "Nigra", T.x cuspidata "Brevifolia" oder T. cuspidata "Spreader"* erhalten wurde.

6. Verfahren nach Anspruch 1, wobei die zweite stationäre Phase neutrales Aluminiumoxid umfasst.

7. Verfahren nach Anspruch 1, wobei die erste, dritte oder vierte stationäre Phase Kieselgel umfasst.

8. Verfahren nach Anspruch 1, wobei das erste, zweite oder dritte Elutionslösungsmittel Aceton, Dichlormethan, Methanol, n-Hexan, Essigsäure-t-butylester, Chloroform oder Essigsäureethylester umfasst.

9. Verfahren nach Anspruch 1, wobei das erste Elutionslösungsmittel Dichlormethan und Methanol umfasst.

10. Verfahren nach Anspruch 1, wobei das zweite Elutionslösungsmittel Aceton umfasst.

11. Verfahren nach Anspruch 1, wobei das dritte Elutionslösungsmittel n-Hexan und Aceton umfasst.

12. Verfahren nach Anspruch 1, wobei das vierte Elutionslösungsmittel Essigsäure-t-butylester, Essigsäure-i-butylester, Ameisensäure-n-butylester, Ameisensäure-i-butylester, Ameisensäure-t-butylester oder Ameisensäure-s-butylester umfasst.

13. Verfahren nach Anspruch 1, wobei das Kristallisationslösungmittel Cyclohexan und Aceton umfasst.

14. Verfahren zum Gewinnen von Paclitaxel aus Paclitaxel enthaltenden Pflanzen, umfassend:
(a) Herstellen eines Paclitaxelextrakts durch Extrahieren von Paclitaxel aus einem Pflanzenmaterial;
(b) Abtrennen von Paclitaxel aus dem Paclitaxelextrakt, umfassend:
(i) Verwendung eines ersten Säulenchromatographiesystems, umfassend eine erste stationäre Phase und ein erstes Elutionslösungsmittel, um mindestens eine Paclitaxel enthaltende Fraktion zu erhalten; und
(ii) Entfernen des ersten Elutionslösungsmittels aus der mindestens einen, in Schritt (b)(i) erhaltenen Fraktion, um einen ersten Rückstand zu bilden;
(c) Abtrennen von Paclitaxel aus dem in Schritt (b)(ii) erhaltenen ersten Rückstand, umfassend:
(i) Verwendung eines zweiten Säulenchromatographiesystems, umfassend eine zweite stationäre Phase und ein zweites Elutionslösungsmittel, um mindestens eine Paclitaxel enthaltende Fraktion aus dem ersten Rückstand zu erhalten; und
(ii) Entfernen des zweiten Elutionslösungsmittels aus der mindestens einen in Schritt (c)(i) erhaltenen Fraktion, um einen zweiten Rückstand zu bilden;
(d) Abtrennen von Paclitaxel aus dem in Schritt (c) (ii) erhaltenen zweiten Rückstand, umfassend:
(i) Verwendung eines dritten Säulenchromatographiesystems, umfassend eine dritte stationäre Phase und ein drittes Elutionslösungsmittel, um mindestens eine Paclitaxel enthaltende Fraktion aus dem zweiten Rückstand zu erhalten; und
(ii) Entfernen des dritten Elutionslösungsmittels aus der mindestens einen in Schritt (d)(i) erhaltenen Fraktion, um einen dritten Rückstand zu bilden;
(e) Abtrennen von Paclitaxel aus dem in Schritt (d)(ii) erhaltenen dritten Rückstand, umfassend:
(i) Verwendung eines vierten Säulenchromatographiesystems, umfassend eine vierte stationäre Phase und ein viertes Elutionslösungsmittel, um mindestens eine Paclitaxel enthaltende Fraktion aus dem dritten Rückstand zu erhalten; und
(ii) Entfernen des vierten Elutionslösungsmittels aus dem dritten, in Schritt (e)(i) erhaltenen Rückstand, um einen vierten Rückstand zu bilden; und
(f) Kristallisieren von Paclitaxel aus dem vierten, in Schritt (e)(ii) erhaltenen Rückstand unter Verwendung eines Kristallisationslösungsmittels, um Paclitaxelkristalle zu erhalten.

15. Verfahren nach Anspruch 14, wobei in Schritt (e) mindestens Cephalomannin aus dem dritten Rückstand entfernt wird.

16. Verfahren nach Anspruch 14, wobei das erste Elutionslösungsmittel durch Unterziehen der mindestens einen in Schritt (b)(i) erhaltenen Fraktion vermindertem Druck entfernt wird.

17. Verfahren nach Anspruch 14, wobei das zweite Elutionslösungsmittel durch Unterziehen der mindestens einen in Schritt (c)(i) erhaltenen Fraktion vermindertem Druck entfernt wird.

18. Verfahren nach Anspruch 14, wobei das dritte Elutionslösungsmittel durch Unterziehen der mindestens einen in Schritt (d)(i) erhaltenen Fraktion vermindertem Druck entfernt wird.

19. Verfahren nach Anspruch 14, wobei das vierte Elutionslösungsmittel durch Unterziehen der mindestens einen in Schritt (e)(i) erhaltenen Fraktion vermindertem Druck entfernt wird.

20. Verfahren nach Anspruch 14, weiterhin umfassend Trocknen der in Schritt (f) erhaltenen Paclitaxelkristalle.

21. Verfahren nach Anspruch 20, wobei die Paclitaxelkristalle unter Vakuum getrocknet werden.

22. Verfahren nach Anspruch 14, wobei das Paclitaxel aus dem Pflanzenmaterial unter Verwendung eines ersten wässrigen Lösungsmittels entfernt wird.

23. Verfahren nach Anspruch 22, wobei das erste wässrige Lösungsmittel Wasser und Aceton umfasst.

24. Verfahren nach Anspruch 14, wobei Schritt (a) bei Raumtemperatur stattfindet.

25. Verfahren nach Anspruch 14, wobei mehr als eine Fraktion in Schritt (b)(i) erhalten wird und mindestens einige der Fraktionen vor dem Entfernen des ersten Elutionslösungsmittels in Schritt (b)(ii) vereinigt werden.

26. Verfahren nach Anspruch 14, wobei mehr als eine Fraktion in Schritt (c)(i) erhalten wird und mindestens einige der Fraktionen vor dem Entfernen des zweiten Elutionslösungsmittels in Schritt (c)(ii) vereinigt werden.

27. Verfahren nach Anspruch 14, wobei mehr als eine Fraktion in Schritt (d)(i) erhalten wird und mindestens einige der Fraktionen vor dem Entfernen des dritten Elutionslösungsmittels in Schritt (d)(ii) vereinigt werden.

28. Verfahren nach Anspruch 14, wobei mehr als eine Fraktion in Schritt (e)(i) erhalten wird und mindestens einige der Fraktionen vor dem Entfernen des vierten Elutionslösungsmittels in Schritt (e)(ii) vereinigt werden.

29. Verfahren nach Anspruch 14, wobei das Kristallisieren von Schritt (f) über einen Zeitraum von 24 Stunden stattfindet.

30. Verfahren nach Anspruch 14, wobei das Kristallisieren von Schritt (f) bei Raumtemperatur stattfindet.

31. Verfahren nach Anspruch 14, wobei das Pflanzenmaterial aus Pflanzenteilen erhalten wird.

32. Verfahren nach Anspruch 31, wobei der Pflanzenmaterialextrakt durch Extrahieren des Pflanzenmaterials aus Pflanzenteilen, umfassend etwa 40 bis etwa 60 Gewicht / Gewicht Prozent oberirdische Teile und etwa 60 bis etwa 40 Gewicht / Gewicht Prozent Wurzeln, hergestellt wird.

33. Verfahren nach Anspruch 14, wobei das Pflanzenmaterial aus *Taxus media, T. x media "Henryi ", T. x media "Runyan", T. cuspidata, T. x media "Halloran", T. x media "Hatfield", T. x media "Hicksii", T. x media "Tauntonii", T. x media "Dark Green Spreader", T. x media "Wardii ", T. x media "Brownii", T.x media "Densiformis", T.x media "Nigra", T.x cuspidata "Brevifolia" oder T. cuspidata "Spreader"* erhaltenes Pflanzenmaterial umfasst.

34. Verfahren nach Anspruch 14, wobei die zweite stationäre Phase neutrales Aluminiumoxid umfasst.

35. Verfahren nach Anspruch 14, wobei die erste, dritte oder vierte stationäre Phase Kieselgel umfasst.

36. Verfahren nach Anspruch 14, wobei das erste, zweite oder dritte Elutionslösungsmittel Aceton, Dichlormethan, Methanol, n-Hexan, Essigsäure-t-butylester, Chloroform oder Essigsäureethylester umfasst.

37. Verfahren nach Anspruch 14, wobei das erste Elutionslösungsmittel Dichlormethan und Methanol umfasst.

38. Verfahren nach Anspruch 14, wobei das zweite Elutionslösungsmittel Aceton umfasst.

39. Verfahren nach Anspruch 14, wobei das dritte Elutionslösungsmittel n-Hexan und Aceton umfasst.

40. Verfahren nach Anspruch 14, wobei das vierte Elutionslösungsmittel Essigsäure-t-butylester, Essigsäure-i-butylester, Ameisensäure-n-butylester, Ameisensäure-i-butylester, Ameisensäure-t-butylester oder Ameisensäure-s-butylester umfasst.

41. Verfahren nach Anspruch 14, wobei das Kristallisationslösungsmittel Cyclohexan und Aceton umfasst.

42. Verfahren zum Gewinnen von Paclitaxel aus *Taxus media,* umfassend:
(a) Herstellen eines Pflanzenmaterialextrakts aus *Taxus media* durch Extrahieren des Pflanzenmaterials mit einem wässrigen Lösungsmittel, umfassend 40 bis 60 Volumen / Volumen Prozent Aceton bei Raumtemperatur;
(b) Herstellen eines Paclitaxelextrakts durch Extrahieren von Paclitaxel aus dem in (a) erhaltenen Pflanzenmaterialextrakt;
(c) Abtrennen von Paclitaxel aus dem Paclitaxelextrakt, umfassend:
(i) Verwendung eines ersten Chromatographiesystems, umfassend Kieselgel als eine stationäre Phase und ein Elutionslösungsmittel, umfassend Dichlormethan allein oder ein Gemisch von Dichlormethan und Methanol, um mindestens eine Paclitaxel enthaltende Fraktion zu erhalten; und
(ii) Unterziehen der mindestens einen in Schritt (c)(i) erhaltenen Fraktion vermindertem Druck, um das Dichlormethan und/oder Methanol zu entfernen;
(d) Abtrennen von Paclitaxel aus der mindestens einen in Schritt (c)(ii) erhaltenen Fraktion, umfassend:
(i) Verwendung eines zweiten Säulenchromatographiesystems, umfassend neutrales Aluminiumoxid als eine stationäre Phase, und ein Elutionslösungsmittel, umfassend Aceton, um mindestens eine Paclitaxel enthaltende Fraktion zu erhalten; und
(ii) Unterziehen der mindestens einen in Schritt (d)(i) erhaltenen Fraktion vermindertem Druck, um das Aceton zu entfernen;
(e) Abtrennen von Paclitaxel aus der mindestens einen in Schritt (d)(ii) erhaltenen Fraktion, umfassend:
(i) Verwendung eines dritten Säulenchromatographiesystems, umfassend Kieselgel als eine stationäre Phase und ein Elutionslösungsmittel, umfassend ein Gemisch von n-Hexan und Aceton, und um mindestens eine Paclitaxel enthaltende Fraktion zu erhalten; und
(ii) Unterziehen der mindestens einen in Schritt (e)(i) erhaltenen Fraktion vermindertem Druck, um das n-Hexan und Aceton zu entfernen;
(f) Abtrennen von Paclitaxel aus der mindestens einen in Schritt (e)(ii) erhaltenen Fraktion, umfassend:
(i) Verwendung eines vierten Säulenchromatographiesystems, umfassend Kieselgel als eine stationäre Phase, und ein Elutionslösungsmittel, umfassend Essigsäure-t-butylester, um mindestens Cephalomannin zu entfernen und um mindestens eine Paclitaxel enthaltende Fraktion zu erhalten; und
(ii) Unterziehen der mindestens einen in Schritt (f)(i) erhaltenen Fraktion vermindertem Druck, um den Essigsäure-t-butylester zu entfernen;
(g) Kristallisieren der mindestens einen in Schritt (f)(ii) erhaltenen Fraktion über einen Zeitraum von 24 Stunden unter Verwendung eines Gemisches von Cyclohexan und Aceton als dem Kristallisationslösungsmittel bei Raumtemperatur, um Paclitaxelkristalle zu erhalten; und
(h) Trocknen der in (g) erhaltenen Paclitaxelkristalle unter Vakuum für 48 Stunden.

43. Verfahren nach Anspruch 42, wobei die Herstellung des Paclitaxelextrakts in Schritt (b) weiterhin umfasst:
(a) Aufkonzentrieren des Pflanzenmaterialextrakts unter vermindertem Druck, um das Aceton zu entfernen, um ein Konzentrat zu bilden;
(b) Verdünnen des Konzentrats mit Methylalkohol;
(c) Extrahieren des Konzentrats mit Dichlormethan, um einen Dichlormethanextrakt zu bilden; und
(d) Aufkonzentrieren des Dichlormethanextrakts unter vermindertem Druck, um den Paclitaxelextrakt zu bilden.

44. Verfahren zum Gewinnen von Paclitaxel aus *Taxus media,* umfassend:
(a) Herstellen eines Pflanzenmaterialextrakts aus *Taxus media* durch Extrahieren des Pflanzenmaterials mit einem wässrigen Lösungsmittel, umfassend etwa 40 bis etwa 60 Volumen / Volumen Prozent Aceton bei Raumtemperatur;
(b) Herstellen eines Paclitaxelextrakts durch Extrahieren von Paclitaxel aus dem in (a) erhaltenen Pflanzenmaterialextrakt;
(c) Abtrennen von Paclitaxel aus dem Paclitaxelextrakt, umfassend:
(i) Verwendung eines ersten Chromatographiesystems, umfassend Kieselgel als eine stationäre Phase und Elutionslösungsmittel, umfassend Dichlormethan und Methanol, um mindestens eine Paclitaxel enthaltende Fraktion zu erhalten;
(ii) Unterziehen der mindestens einen in Schritt (c)(i) erhaltenen Fraktion vermindertem Druck, um einen Rückstand zu erhalten, und
(iii) Auflösen des in Schritt (c)(ii) erhaltenen Rückstands in Aceton, um eine Aceton-Rückstandszusammensetzung zu erhalten;
(d) Abtrennen von Paclitaxel aus der in Schritt (c)(iii) erhaltenen Aceton-Rückstandszusammensetzung, umfassend:
(i) Verwendung eines zweiten Säulenchromatographiesystems, umfassend neutrales Aluminiumoxid als eine stationäre Phase und ein Elutionslösungsmittel, umfassend Aceton, um mindestens eine Paclitaxel enthaltende Fraktion zu erhalten; und
(ii) Unterziehen der mindestens einen in Schritt (d)(i) erhaltenen Fraktion vermindertem Druck, um das Aceton zu entfernen,
(e) Abtrennen von Paclitaxel aus der mindestens einen in Schritt (d)(ii) erhaltenen Fraktion, umfassend:
(i) Verwendung eines dritten Säulenchromatographiesystems, umfassend Kieselgel als eine stationäre Phase und ein Elutionslösungsmittel, umfassend n-Hexan und Aceton, um mindestens eine Paclitaxel enthaltende Fraktion zu erhalten; und
(ii) Unterziehen der mindestens einen in Schritt (e)(i) erhaltenen Fraktion vermindertem Druck, um das n-Hexan und Aceton zu entfernen;
(f) Abtrennen von Paclitaxel aus der mindestens einen in Schritt (e)(ii) erhaltenen Fraktion, umfassend:
(i) Verwendung eines vierten Säulenchromatographiesystems, umfassend Kieselgel als eine stationäre Phase, und ein Elutionslösungsmittel, umfassend Essigsäure-t-butylester, um mindestens Cephalomannin zu entfernen und um mindestens eine Paclitaxel enthaltende Fraktion zu erhalten; und
(ii) Unterziehen der mindestens einen in Schritt (f)(i) erhaltenen Fraktion vermindertem Druck, um den Essigsäure-t-butylester zu entfernen;
(g) Kristallisieren der mindestens einen in Schritt (f)(ii) erhaltenen Fraktion über einen Zeitraum von 24 Stunden unter Verwendung eines Gemisches von Cyclohexan und Aceton als dem Kristallisationslösungsmittel bei Raumtemperatur, um Paclitaxelkristalle zu erhalten; und
(h) Trocknen der in (g) erhaltenen Paclitaxelkristalle unter Vakuum für 48 Stunden.

45. Verfahren nach Anspruch 44, wobei der *Taxus media T. media Hicksii* umfasst.

46. Verfahren zum Gewinnen von Paclitaxel aus *Taxus media,* umfassend:
(a) Herstellen eines Pflanzenmaterialextrakts aus *Taxus media* durch Extrahieren des Pflanzenmaterials mit einer wässrigen Lösung, umfassend etwa 40 bis etwa 60 Volumen / Volumen Prozent Aceton bei Raumtemperatur;
(b) Herstellen eines Paclitaxelextrakts durch Extrahieren von Paclitaxel aus dem in (a) erhaltenen Pflanzenmaterialextrakt;
(c) Abtrennen von Paclitaxel aus dem Paclitaxelextrakt, umfassend:
(i) Verwendung eines ersten Chromatographiesystems, umfassend neutrales Aluminiumoxid als eine.stationäre Phase und ein Elutionslösungsmittel, umfassend Dichlormethan und Methanol, um mindestens eine Paclitaxel enthaltende Fraktion zu erhalten; und
(ii) Unterziehen der mindestens einen in Schritt (c)(i) erhaltenen Fraktion vermindertem Druck, um das Dichlormethan und Methanol zu entfernen;
(d) Abtrennen von Paclitaxel aus der mindestens einen, in Schritt (c)(ii) erhaltenen Fraktion, umfassend:
(i) Verwendung eines zweiten Chromatographiesystems, umfassend Kieselgel als eine stationäre Phase und ein Elutionslösungsmittel, umfassend Dichlormethan und Methanol, um mindestens eine Paclitaxel enthaltende Fraktion zu erhalten;
(ii) Unterziehen der mindestens einen in Schritt (d)(i) erhaltenen Fraktion vermindertem Druck, um einen Rückstand zu erhalten, und
(iii) Auflösen des in Schritt (d)(ii) erhaltenen Rückstands in Aceton, um eine Aceton-Rückstandszusammensetzung zu erhalten;
(e) Abtrennen von Paclitaxel aus der in Schritt (d) (iii) erhaltenen Aceton-Rückstandszusammensetzung, umfassend:
(i) Verwendung eines dritten Säulenchromatographiesystems, umfassend Kieselgel als eine stationäre Phase und ein Elutionslösungsmittel, umfassend n-Hexan und Aceton, um mindestens eine Paclitaxel enthaltende Fraktion zu erhalten; und
(ii) Unterziehen der mindestens einen in Schritt (e)(i) erhaltenen Fraktion vermindertem Druck, um das Gemisch von n-Hexan und Aceton zu entfernen;
(f) Abtrennen von Paclitaxel aus der mindestens einen in Schritt (e)(ii) erhaltenen Fraktion, umfassend:
(i) Verwendung eines vierten Säulenchromatographiesystems, umfassend Kieselgel als eine stationäre Phase und ein Elutionslösungsmittel, umfassend Essigsäure-t-butylester, um mindestens Cephalomannin zu entfernen und um mindestens eine Paclitaxel enthaltende Fraktion zu erhalten; und
(ii) Unterziehen der mindestens einen in Schritt (f)(i) erhaltenen Fraktion vermindertem Druck, um den Essigsäure-t-butylester zu entfernen;
(g) Kristallisieren der mindestens einen in Schritt (f)(ii) erhaltenen Fraktion über einen Zeitraum von 24 Stunden unter Verwendung eines Gemisches von Cyclohexan und Aceton als dem Kristallisationslösungsmittel bei Raumtemperatur, um Paclitaxelkristalle zu erhalten; und
(h) Trocknen der in (g) erhaltenen Paclitaxelkristalle unter Vakuum für 48 Stunden.

47. Verfahren nach Anspruch 46, wobei der *Taxus media T. media "Dark Green Spreader"* umfasst.

## Revendications

1. Procédé permettant d'obtenir du paclitaxel à partir de plantes contenant du paclitaxel comprenant :
(a) de préparer un extrait de paclitaxel par extraction de paclitaxel à partir d'un matériel végétal ;
(b) de séparer le paclitaxel de l'extrait de paclitaxel en utilisant un premier système de chromatographie sur colonne comprenant une première phase stationnaire et un premier solvant d'élution pour obtenir au moins une fraction contenant du paclitaxel ;
(c) de séparer le paclitaxel de ladite au moins une fraction obtenue dans l'étape (b) en utilisant un deuxième système de chromatographie sur colonne comprenant une deuxième phase stationnaire et un deuxième solvant d'élution pour obtenir au moins une fraction contenant du paclitaxel ;
(d) de séparer le paclitaxel de ladite au moins une fraction obtenue dans l'étape (c) en utilisant un troisième système de chromatographie sur colonne comprenant une troisième phase stationnaire et un troisième solvant d'élution pour obtenir au moins une fraction contenant du paclitaxel ;
(e) de séparer le paclitaxel de ladite au moins une fraction obtenue dans l'étape (d) en utilisant un quatrième système de chromatographie sur colonne comprenant une quatrième phase stationnaire et un quatrième solvant d'élution pour éliminer au moins la céphalomannine et pour obtenir au moins une fraction contenant du paclitaxel ; et
(f) de cristalliser le paclitaxel contenu dans ladite au moins une fraction obtenue dans l'étape (e) en utilisant un solvant de cristallisation pour obtenir des cristaux de paclitaxel.

2. Procédé selon la revendication 1, comprenant en outre de sécher les cristaux de paclitaxel obtenus dans l'étape (f).

3. Procédé selon la revendication 1, dans lequel le matériel végétal est obtenu à partir de parties de plante.

4. Procédé selon la revendication 3, dans lequel l'extrait de matériel végétal est préparé par extraction du matériel végétal comprenant 40 à 60 pourcent p/p de parties aériennes et 60 à 40 pourcent p/p de racines.

5. Procédé selon la revendication 1, dans lequel le matériel végétal comprend du matériel végétal obtenu à partir de *Taxus media, T.x media « Henryi », T.x media « Runyan », T. cuspidata, T.x media « Halloran », T.x media « Hatfield », T.x media « Hicksii », T.x media « Tauntonii », T.x media « Dark Green Spreader », T.x media* « *Wardii* », *T.x media* « *Brownii* », *T.x media* « *Densiformis* », *T.x media* « *Nigra* », *T.x cuspidata* « *Brevifolia* », ou *T. cuspidata* « *Spreader* ».

6. Procédé selon la revendication 1, dans lequel la deuxième phase stationnaire comprend de l'alumine neutre.

7. Procédé selon la revendication 1, dans lequel la première, la troisième, ou la quatrième phase stationnaire comprend du gel de silice.

8. Procédé selon la revendication 1, dans lequel le premier, le deuxième, ou le troisième solvant d'élution comprend de l'acétone, du dichlorométhane, du méthanol, du n-hexane, de l'acétate de t-butyle, du chloroforme ou de l'acétate d'éthyle.

9. Procédé selon la revendication 1, dans lequel le premier solvant d'élution comprend du dichlorométhane ou du méthanol.

10. Procédé selon la revendication 1, dans lequel le deuxième solvant d'élution comprend de l'acétone.

11. Procédé selon la revendication 1, dans lequel le troisième solvant d'élution comprend du n-hexane ou de l'acétone.

12. Procédé selon la revendication 1, dans lequel le quatrième solvant d'élution comprend de l'acétate de t-butyle, de l'acétate de i-butyle, du formiate de n-butyle, du formiate de i-butyle, du formiate de t-butyle, ou du formiate de s-butyle.

13. Procédé selon la revendication 1, dans lequel le solvant de cristallisation comprend du cyclohexane et de l'acétone.

14. Procédé permettant d'obtenir du paclitaxel à partir de plantes contenant du paclitaxel comprenant :
(a) de préparer un extrait de paclitaxel par extraction de paclitaxel à partir d'un matériel végétal ;
(b) de séparer le paclitaxel de l'extrait de paclitaxel comprenant :
(i) d'utiliser un premier système de chromatographie sur colonne comprenant une première phase stationnaire et un premier solvant d'élution pour obtenir au moins une fraction contenant du paclitaxel, et
(ii) d'éliminer le premier solvant d'élution de ladite au moins une fraction obtenue dans l'étape (b)(i) pour former un premier résidu ;
(c) de séparer le paclitaxel du premier résidu obtenu dans l'étape (b)(ii) comprenant :
(i) d'utiliser un deuxième système de chromatographie sur colonne comprenant une deuxième phase stationnaire et un deuxième solvant d'élution pour obtenir au moins une fraction contenant du paclitaxel provenant du premier résidu, et
(ii) d'éliminer le deuxième solvant d'élution de ladite au moins une fraction obtenue dans l'étape (c)(i) pour former un deuxième résidu ;
(d) de séparer le paclitaxel du deuxième résidu obtenu dans l'étape (c)(ii) comprenant :
(i) d'utiliser un troisième système de chromatographie sur colonne comprenant une troisième phase stationnaire et un troisième solvant d'élution pour obtenir au moins une fraction contenant du paclitaxel provenant du deuxième résidu, et
(ii) d'éliminer le troisième solvant d'élution de ladite au moins une fraction obtenue dans l'étape (d)(i) pour former un troisième résidu ;
(e) de séparer le paclitaxel du troisième résidu obtenu dans l'étape (d)(ii) comprenant :
(i) d'utiliser un quatrième système de chromatographie sur colonne comprenant une quatrième phase stationnaire et un quatrième solvant pour obtenir au moins une fraction contenant du paclitaxel provenant du troisième résidu, et
(ii) d'éliminer le quatrième solvant d'élution du troisième résidu obtenu dans l'étape (e)(i) pour former un quatrième résidu ; et
(f) de cristalliser le paclitaxel provenant du quatrième résidu obtenu dans l'étape (e)(ii) en utilisant un solvant de cristallisation pour obtenir des cristaux de paclitaxel.

15. Procédé selon la revendication 14, dans lequel dans l'étape (e) au moins de la céphalomannine est éliminée du troisième résidu.

16. Procédé selon la revendication 14, dans lequel le premier solvant d'élution est éliminé en soumettant ladite au moins une fraction obtenue dans l'étape (b)(i) à une pression réduite.

17. Procédé selon la revendication 14, dans lequel le deuxième solvant d'élution est éliminé en soumettant ladite au moins une fraction obtenue dans l'étape (c)(i) à une pression réduite.

18. Procédé selon la revendication 14, dans lequel le troisième solvant d'élution est éliminé en soumettant ladite au moins une fraction obtenue dans l'étape (d)(i) à une pression réduite.

19. Procédé selon la revendication 14, dans lequel le quatrième solvant d'élution est éliminé en soumettant ladite au moins une fraction obtenue dans l'étape (e)(i) à une pression réduite.

20. Procédé selon la revendication 14, comprenant en outre de sécher les cristaux de paclitaxel obtenus dans l'étape (f).

21. Procédé selon la revendication 20, dans lequel les cristaux de paclitaxel sont séchés sous vide.

22. Procédé selon la revendication 14, dans lequel le paclitaxel est extrait du matériel végétal en utilisant un premier solvant aqueux.

23. Procédé selon la revendication 22, dans lequel le premier solvant aqueux comprend de l'eau et de l'acétone.

24. Procédé selon la revendication 14, dans lequel l'étape (a) se produit à température ambiante.

25. Procédé selon la revendication 14, dans lequel plus d'une fraction est obtenue dans l'étape (b)(i) et au moins certaines des fractions sont combinées avant élimination du premier solvant d'élution dans l'étape (b)(ii).

26. Procédé selon la revendication 14, dans lequel plus d'une fraction est obtenue dans l'étape (c)(i) et au moins certaines des fractions sont combinées avant élimination du deuxième solvant d'élution dans l'étape (c)(ii).

27. Procédé selon la revendication 14, dans lequel plus d'une fraction est obtenue dans l'étape (d)(i) et au moins certaines des fractions sont combinées avant élimination du troisième solvant d'élution dans l'étape (d)(ii).

28. Procédé selon la revendication 14, dans lequel plus d'une fraction est obtenue dans l'étape (e)(i) et au moins certaines des fractions sont combinées avant élimination du quatrième solvant d'élution dans l'étape (e)(ii).

29. Procédé selon la revendication 14, dans lequel la cristallisation de l'étape (f) se produit sur une période de 24 heures.

30. Procédé selon la revendication 14, dans lequel la cristallisation de l'étape (f) se produit à température ambiante.

31. Procédé selon la revendication 14, dans lequel le matériel végétal est obtenu à partir de parties de plante.

32. Procédé selon la revendication 31, dans lequel l'extrait de matériel végétal est préparé par extraction du matériel végétal à partir de parties de plante comprenant environ 40 à environ 60 pourcent p/p de parties aériennes et environ 60 à environ 40 pourcent p/p de racines.

33. Procédé selon la revendication 14, dans lequel le matériel végétal comprend du matériel végétal obtenu à partir de *Taxus media, T.x media « Henryi* », *T.x media « Runyan », T. cuspidata, T.x media « Halloran », T.x media « Hatfield », T.x media « Hicksii », T.x media « Tauntonii », T.x media « Dark Green Spreader », T.x media « Wardii », T.x media « Brownii », T.x media « Densiformis », T.x media « Nigra », T.x cuspidata « Brevifolia »,* ou *T. cuspidata « Spreader ».*

34. Procédé selon la revendication 14, dans lequel la deuxième phase stationnaire comprend de l'alumine neutre.

35. Procédé selon la revendication 14, dans lequel la première, la troisième, ou la quatrième phase stationnaire comprend du gel de silice.

36. Procédé selon la revendication 14, dans lequel le premier, le deuxième, ou le troisième solvant d'élution comprend de l'acétone, du dichlorométhane, du méthanol, du n-hexane, de l'acétate de t-butyle, du chloroforme, ou de l'acétate d'éthyle.

37. Procédé selon la revendication 14, dans lequel le premier solvant d'élution comprend du dichlorométhane et du méthanol.

38. Procédé selon la revendication 14, dans lequel le deuxième solvant d'élution comprend de l'acétone.

39. Procédé selon la revendication 14, dans lequel le troisième solvant d'élution comprend du n-hexane et de l'acétone.

40. Procédé selon la revendication 14, dans lequel le quatrième solvant d'élution comprend de l'acétate de t-butyle, de l'acétate de i-butyle, du formiate de n-butyle, du formiate de i-butyle, du formiate de t-butyle, ou du formiate de s-butyle.

41. Procédé selon la revendication 14, dans lequel le solvant de cristallisation comprend du cyclohexane et de l'acétone.

42. Procédé permettant d'obtenir du paclitaxel à partir de *Taxus media* comprenant :
(a) de préparer un extrait de matériel végétal à partir de *Taxus media* par extraction du matériel végétal avec un solvant aqueux comprenant 40 à 60 pourcent v/v d'acétone à température ambiante ;
(b) de préparer un extrait de paclitaxel par extraction de paclitaxel à partir de l'extrait de matériel végétal obtenu dans (a) ;
(c) de séparer le paclitaxel de l'extrait de paclitaxel comprenant :
(i) d'utiliser un premier système de chromatographie sur colonne comprenant un gel de silice comme phase stationnaire et un solvant d'élution comprenant du dichlorométhane seul ou un mélange de dichlorométhane et de méthanol pour obtenir au moins une fraction contenant du paclitaxel, et
(ii) de soumettre ladite au moins une fraction obtenue dans l'étape (c)(i) à une pression réduite pour éliminer le dichlorométhane et/ou le méthanol ;
(d) de séparer le paclitaxel de ladite au moins une fraction obtenue dans l'étape (c)(ii) comprenant :
(i) d'utiliser un deuxième système de chromatographie sur colonne comprenant de l'alumine neutre comme phase stationnaire et un solvant d'élution comprenant de l'acétone pour obtenir au moins une fraction contenant du paclitaxel, et
(ii) de soumettre ladite au moins une fraction obtenue dans l'étape (d)(i) à une pression réduite pour éliminer l'acétone ;
(e) de séparer le paclitaxel de ladite au moins une fraction obtenue dans l'étape (d)(ii) comprenant :
(i) d'utiliser un troisième système de chromatographie sur colonne comprenant un gel de silice comme phase stationnaire et un solvant d'élution comprenant un mélange de n-hexane et d'acétone pour obtenir au moins une fraction contenant du paclitaxel, et
(ii) de soumettre ladite au moins une fraction obtenue dans l'étape (e)(i) à une pression réduite pour éliminer le n-hexane et l'acétone ;
(f) de séparer le paclitaxel de ladite au moins une fraction obtenue dans l'étape (e)(ii) comprenant :
(i) d'utiliser un quatrième système de chromatographie sur colonne comprenant un gel de silice comme phase stationnaire et un solvant d'élution comprenant de l'acétate de t-butyle pour éliminer au moins la céphalomannine et pour obtenir au moins une fraction contenant du paclitaxel, et
(ii) de soumettre ladite au moins une fraction obtenue dans l'étape (f)(i) à une pression réduite pour éliminer l'acétate de t-butyle ;
(g) de cristalliser ladite au moins une fraction obtenue dans l'étape (f)(ii) sur une période de 24 heures à l'aide d'un mélange de cyclohexane et d'acétone comme solvant de cristallisation à température ambiante pour obtenir des cristaux de paclitaxel ; et
(h) de sécher les cristaux de paclitaxel obtenus dans (g) sous vide pendant 48 heures.

43. Procédé selon la revendication 42, dans lequel la préparation de l'extrait de paclitaxel dans l'étape (b) comprend en outre :
(a) de concentrer l'extrait de matériel végétal sous pression réduite pour éliminer l'acétone pour former un concentré ;
(b) de diluer le concentré avec de l'alcool méthylique ;
(c) d'extraire le concentré avec du dichlorométhane pour former un extrait de dichlorométhane ; et
(d) de concentrer l'extrait de dichlorométhane sous pression réduite pour former l'extrait de paclitaxel.

44. Procédé permettant d'obtenir du paclitaxel à partir de *Taxus media* comprenant :
(a) de préparer un extrait de matériel végétal à partir de *Taxus media* par extraction du matériel végétal avec un solvant aqueux comprenant environ 40 à environ 60 pourcent v/v d'acétone à température ambiante ;
(b) de préparer un extrait de paclitaxel par extraction de paclitaxel à partir de l'extrait de matériel végétal obtenu dans (a) ;
(c) de séparer le paclitaxel de l'extrait de paclitaxel comprenant :
(i) d'utiliser un premier système de chromatographie sur colonne comprenant un gel de silice comme phase stationnaire et un solvant d'élution comprenant du dichlorométhane et du méthanol pour obtenir au moins une fraction contenant du paclitaxel,
(ii) de soumettre ladite au moins une fraction obtenue dans l'étape (c)(i) à une pression réduite pour obtenir un résidu, et
(iii) de dissoudre le résidu obtenu dans l'étape (c)(ii) dans de l'acétone pour obtenir une composition acétone-résidu ;
(d) de séparer le paclitaxel de la composition acétone-résidu obtenu dans l'étape (c)(iii) comprenant :
(i) d'utiliser un deuxième système de chromatographie sur colonne comprenant de l'alumine neutre comme phase stationnaire et un solvant d'élution comprenant de l'acétone pour obtenir au moins une fraction contenant du paclitaxel, et
(ii) de soumettre ladite au moins une fraction obtenue dans l'étape (d)(i) à une pression réduite pour éliminer l'acétone ;
(e) de séparer le paclitaxel de ladite au moins une fraction obtenue dans l'étape (d)(ii) comprenant :
(i) d'utiliser un troisième système de chromatographie sur colonne comprenant un gel de silice comme phase stationnaire et un solvant d'élution comprenant du n-hexane et de l'acétone pour obtenir au moins une fraction contenant du paclitaxel, et
(ii) de soumettre ladite au moins une fraction obtenue dans l'étape (e)(i) à une pression réduite pour éliminer le n-hexane et l'acétone ;
(f) de séparer le paclitaxel de ladite au moins une fraction obtenue dans l'étape (e)(ii) comprenant :
(i) d'utiliser un quatrième système de chromatographie sur colonne comprenant un gel de silice comme phase stationnaire et un solvant d'élution comprenant de l'acétate de t-butyle pour éliminer au moins la céphalomannine et pour obtenir au moins une fraction contenant du paclitaxel, et
(ii) de soumettre ladite au moins une fraction obtenue dans l'étape (f)(i) à une pression réduite pour éliminer l'acétate de t-butyle ;
(g) de cristalliser ladite au moins une fraction obtenue dans l'étape (f)(ii) sur une période de 24 heures à l'aide d'un mélange de cyclohexane et d'acétone comme solvant de cristallisation à température ambiante pour obtenir des cristaux de paclitaxel ; et
(h) de sécher les cristaux de paclitaxel obtenus dans (g) sous vide pendant 48 heures.

45. Procédé selon la revendication 44, dans lequel *Taxus media* comprend *T. media Hicksii.*

46. Procédé permettant d'obtenir du paclitaxel à partir de *Taxus media* comprenant :
(a) de préparer un extrait de matériel végétal à partir de *Taxus media* par extraction du matériel végétal avec un solvant aqueux comprenant environ 40 à environ 60 pourcent v/v d'acétone à température ambiante ;
(b) de préparer un extrait de paclitaxel par extraction de paclitaxel à partir de l'extrait de matériel végétal obtenu dans (a) ;
(c) de séparer le paclitaxel de l'extrait de paclitaxel comprenant :
(i) d'utiliser un premier système de chromatographie sur colonne comprenant de l'alumine neutre comme phase stationnaire et un solvant d'élution comprenant du dichlorométhane et du méthanol pour obtenir au moins une fraction contenant du paclitaxel, et
(ii) de soumettre ladite au moins une fraction obtenue dans l'étape (c)(i) à une pression réduite pour éliminer le dichlorométhane et le méthanol ;
(d) de séparer le paclitaxel de la ladite au moins une fraction obtenue dans l'étape (c)(ii) comprenant :
(i) d'utiliser un deuxième système de chromatographie sur colonne comprenant un gel de silice comme phase stationnaire et un solvant d'élution comprenant du dichlorométhane et du méthanol pour obtenir au moins une fraction contenant du paclitaxel,
(ii) de soumettre ladite au moins une fraction obtenue dans l'étape (d)(i) à une pression réduite pour obtenir un résidu ; et
(iii) de dissoudre le résidu obtenu dans l'étape (d)(ii) dans de l'acétone pour obtenir une composition acétone-résidu ;
(e) de séparer le paclitaxel de la composition acétone-résidu obtenue dans l'étape (d)(iii) comprenant :
(i) d'utiliser un troisième système de chromatographie sur colonne comprenant un gel de silice comme phase stationnaire et un solvant d'élution comprenant du n-hexane et de l'acétone pour obtenir au moins une fraction contenant du paclitaxel, et
(ii) de soumettre ladite au moins une fraction obtenue dans l'étape (e)(i) à une pression réduite pour éliminer le mélange de n-hexane et d'acétone ;
(f) de séparer le paclitaxel de ladite au moins une fraction obtenue dans l'étape (e)(ii) comprenant :
(i) d'utiliser un quatrième système de chromatographie sur colonne comprenant un gel de silice comme phase stationnaire et un solvant d'élution comprenant de l'acétate de t-butyle pour éliminer au moins la céphalomannine et pour obtenir au moins une fraction contenant du paclitaxel, et
(ii) de soumettre ladite au moins une fraction obtenue dans l'étape (f)(i) à une pression réduite pour éliminer l'acétate de t-butyle ;
(g) de cristalliser ladite au moins une fraction obtenue dans l'étape (f)(ii) sur une période de 24 heures à l'aide d'un mélange de cyclohexane et d'acétone comme solvant de cristallisation à température ambiante pour obtenir des cristaux de paclitaxel ; et
(h) de sécher les cristaux de paclitaxel obtenus dans (g) sous vide pendant 48 heures.

47. Procédé selon la revendication 46, dans lequel *Taxus media* comprend *T. media « dark green spreader* ».
